# EUROPEAN PATENT SPECIFICATION

(11) **EP 1 208 859 B1**
(45) Date of publication and mention of the grant of the patent: **14.03.2007**
(21) Application number: 01126592.3
(22) Date of filing: 16.11.2001
(51) Int. Cl.: A61M 5/32

(54) **Safety syringe**
Sicherheitsspritze
Seringue de sécurité

(30) Priority: 16.11.2000 JP 2000349009
(43) Date of publication of application: 29.05.2002
(73) Proprietor: JMS Co., Ltd., Hiroshima-shi, Hiroshima 730-8652 (JP)
(72) Inventor: Kunishige, Takahiko, JMS Company Limited, Hiroshima-shi, Hiroshima 730-8652 (JP)
(74) Representative: Müller-Boré & Partner Patentanwälte

(56) References cited:
- EP-A- 0 962 230
- EP-A- 1 075 850
- FR-A- 2 530 473
- US-A- 5 501 670
- US-A- 6 010 486

## Description

The present invention relates to a disposable syringe equipped with a faulty stick prevention mechanism in which a needle can be retracted within the barrel main body.

Conventionally, when a healthcare worker at a medical site uses a syringe for a blood test, etc., after use the needle is stored within a cover having an elongated cylindrical shape, etc., but there are cases in which the workers accidentally stick themselves with the needle. This not only causes pain but also raises the possibility that, if blood adhering to the needle originates from a patient infected with a virus, etc., the healthcare worker might be infected with the virus, etc. via the blood. There have therefore been developed a large number of syringes, etc. having a mechanism for preventing accidental sticks.
JP, A, 7-51372 and JP, A, 11-89933 disclose a disposable syringe formed from a cylindrical main body and a piston, the cylindrical main body being provided with a needle-connecting end piece at one end and a piston-receiving aperture at the other end, characterised in that the cylindrical main body includes a needle that is retractable within said cylindrical main body, a hub having one end thereof connected to the tip of the needle, and abutment legs provided on at least one pair of projections extending from the other end of the hub, and the piston being characterised in that it has a blind hole on one end that faces the needle, the ends of said at least one pair of projections being allowed to enter the blind hole, and a locking member that can engage with said at least one pair of projections.
The above-mentioned publications disclose a syringe wherein, when the aforementioned piston reaches the needle end of the cylindrical main body on completion of the injection, the ends of said at least one pair of projections provided on the needle-connecting end piece of the cylindrical member engage with the locking member provided on the extremity of the piston, and the operation of pulling back the piston retracts the connected needle within the cylindrical member, thereby dispensing with the unsafe operation in the vicinity of the needle, and allowing the needle to be safely removed.
JP, A, 9-501078 discloses a syringe having a similar mechanism for preventing accidental sticks. The syringe disclosed in the above-mentioned publication has a mechanism in which a hole that captures the base of a needle is provided within a gasket provided on the extremity of a piston, the hole is equipped with a drop out prevention tab, a click sound is made when the needle engages with the tab, and the engagement is thus recognised by aural indication means.
In the syringes disclosed in JP, A, 7-51372 and JP, A, 11-89933, visual confirmation is required for completion of the engagement of the projections on the needle base with the locking member on the piston extremity when the injection is completed. However, since the projections on the needle base and the locking member on the piston extremity are placed within the extremity of the barrel, it cannot be said that visual confirmation is easy.
On the other hand, the syringe disclosed in JP, A, 9-501078 has a mechanism in which completion of the operation of engaging the needle with the extremity of the piston when the injection is completed can be recognised by aural indication means. However, since both the members that are to be engaged with each other, that is to say, the pair of projections and the locking member, are very small, it is essentially impossible to generate an aural indication that can be recognised, and good operability cannot necessarily be obtained. In such a syringe, in order to engage both the members more reliably, the piston might be depressed with a force that is stronger than that for usual discharge operation, and there is thereby a possibility that pain might be inflicted on the human body to which the injection is being given. Furthermore, when the engagement between the two members is insufficient, the needle cannot be retracted within the barrel main body by pulling back the piston, and it is necessary to engage the two members by depressing the piston again, thereby making the operation complicated. Moreover, when the piston is re-depressed there is a risk of a faulty needle-stick to the human body.

US 5,501,670 discloses a syringe utilizing a cartridge piston having, an interlocking needle receiving cup and retraction cage structure graspable by a plunger hook, and a break-away needle assembly in order to provide simple and reliable retraction of a needle cannula into a disposable medicament cartridge.

US 6,010,486 discloses a retracting needle syringe providing a syringe barrel for holding a liquid and a needle that is fixably attached to an inner hub having frangible portions. On completion of the discharge of the liquid a plunger tip of a plunger rod, which is slidable within barrel, is pushed against the inner hub so as to break off the frangible portions from the inner hub. Following that the frangible portions are removed from the inner hub, a spring compressed and disposed over the inner hub expands and moves the inner hub including the needle into the syringe.

It is the object of the invention to provide a syringe that allows a needle to be retracted within a barrel main body by providing a recognisable signal that clearly indicates the engagement of the needle base and the piston. Further, it is another object of the invention to provide means to generate such a signal.

This object is fulfilled by a syringe having the features as disclosed in claim 1. Preferred embodiments are defined in the dependent subclaims.

A syringe that allows a needle to be retracted within a barrel main body, on completion of the discharge of a liquid filling the syringe, by engaging a needle base provided on an extremity of the barrel main body with an extremity of a piston and pulling back the piston, wherein signal generating means for generating a notice signal to provide a notification of completion of the engagement between the needle base and the extremity of the piston is provided as projections provided on the inner face of the barrel main body and on the side of the piston, and the notice signal is generated by physical contact of the two projections accompanying sliding of the piston.

Accordingly, there is proposed a syringe that allows a needle to be retracted within the barrel main body on completion of the injection by engaging a needle base provided on an extremity of the syringe with an extremity of the piston and pulling back the piston, characterised in that, in order to allow the engagement between the needle base and the piston extremity to be easily and reliably recognised, signal generating means is provided in a position other than the position where the respective engagement means engage, it being difficult to generate a distinct notice signal at the position where the respective engagement means engage. Furthermore, in accordance with second and third aspects of the present invention, the notice signal is an aurally recognisable signal and/or a tactilely recognisable signal. Furthermore, in accordance with a fourth aspect of the present invention, a projection provided on the inner face of the barrel main body as the signal generating means is an annular projection, and in accordance with a fifth aspect, a projection provided on the side of the piston as the signal generating means is a plate-like projection, characterised in that the aforementioned projections are made to physically contact with each other as the piston slides and the plate-like projection snaps back, thereby distinctively generating each of the aforementioned notice signals. It is thereby possible to easily and reliably recognise the engagement between the needle base and the piston extremity.
In the present specification, the terms "extremity" and "base" are used as terms expressing positional relationships; the term "extremity" in each of the syringe components is defined as the needle side in the axial direction, that is to say, in the case of the barrel main body, the side where a nozzle extremity is positioned, and in the case of the piston, the side where its gasket is positioned. On the other hand, the term "base" is defined as the opposite side in the axial direction, that is to say, in the case of the barrel main body, the side where its flange is positioned, and in the case of the piston, the side where its knob is positioned.

In the drawings:
FIG. 1 is a front perspective view of a syringe in accordance with the present invention, which is filled with a liquid content;
FIG. 2 is a front perspective view of the syringe in accordance with the present invention when a plate-like projection comes into contact with an annular projection when the liquid content is discharged;
FIG. 3 is a front perspective view of the syringe in accordance with the present invention in a state in which the plate-like projection is bent due to a stress being applied thereto when the liquid content is being discharged;
FIG. 4 is a front perspective view of the syringe in accordance with the present invention in a state in which, when the stress applied to the plate-like projection during discharge of the liquid content has been released, its original shape is restored, and at the same time the discharge of the liquid content in the syringe is completed;
FIG. 5 is a front perspective view of the syringe in accordance with the present invention when its needle is retracted within its barrel main body;
FIGS. 6 (a) and (b) are magnified cross sections of the extremity of the syringe in accordance with the present invention;
FIG. 7 (a) is a front view of a piston in accordance with the present invention;
FIG. 7 (b) is a top view of the piston in accordance with the present invention;
FIG. 8 (a) is a front view of a piston in accordance with another embodiment of the present invention;
FIG. 8 (b) is a top view of the piston in accordance with another embodiment of the present invention;
FIGS. 9 (a), (b) and (c) are magnified views (cross sections at line X-X' in FIG. 7 (a)) of plate-like projections of the piston in accordance with embodiments of the present invention.

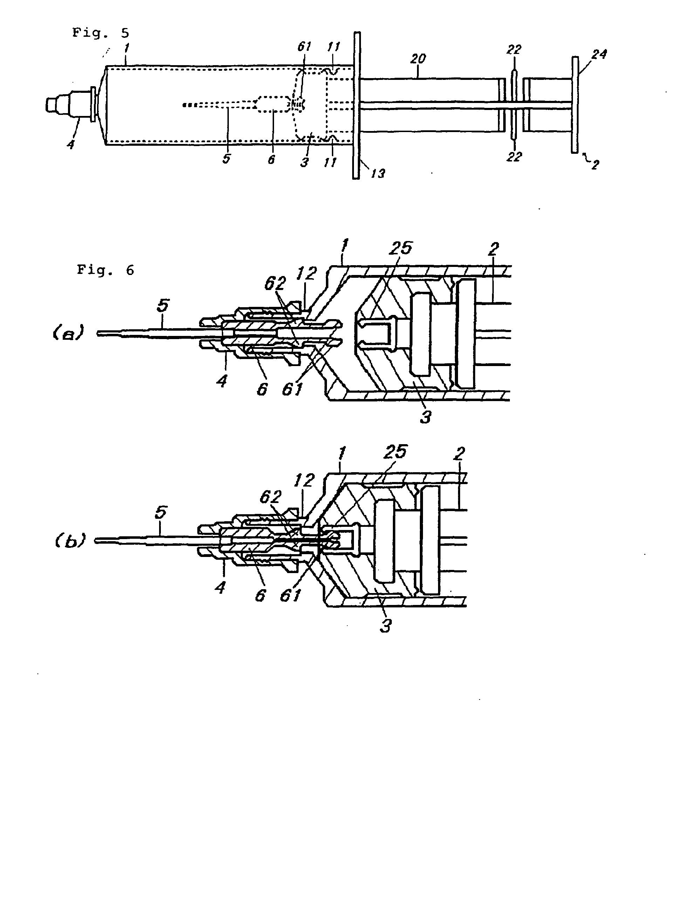

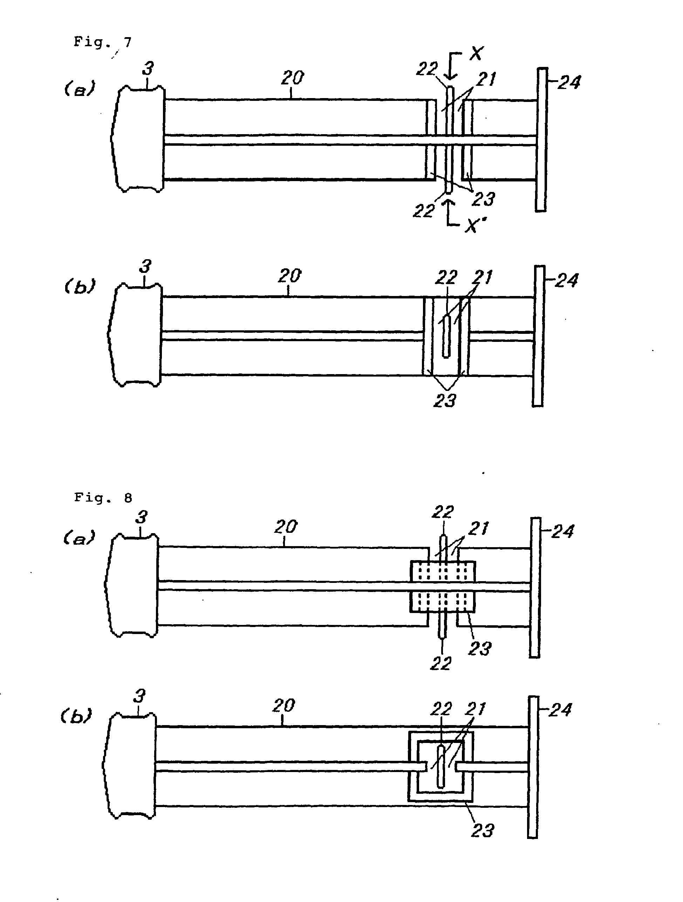

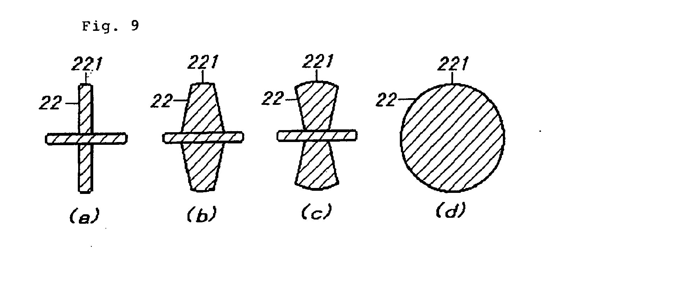

A syringe of the present invention is explained in detail below by reference to the attached drawings. Firstly referring to FIG. 6 (a), an outline of the constitution is explained. The syringe in accordance with the present invention is made of a transparent resin and is formed from a hollow tubular barrel main body 1, a needle 5 that latches onto the extremity of the barrel main body 1 and has on its base engagement means 61, and a piston 2 that has on its extremity engagement means 25 and reciprocates within the barrel main body 1, the engagement means 25 engaging with the above-mentioned engagement means 61. The materials of the barrel main body 1 and the piston 2 are desirably of a synthetic resin that is similar to that of a typical syringe. In addition, in order for projections, which are a characteristic of the present invention, to generate an aural signal and/or a tactile signal, it is necessary to use a material that has both moderate rigidity and moderate elasticity. Specific examples of the preferable material include synthetic resins such as polypropylene and polymethylpentene.

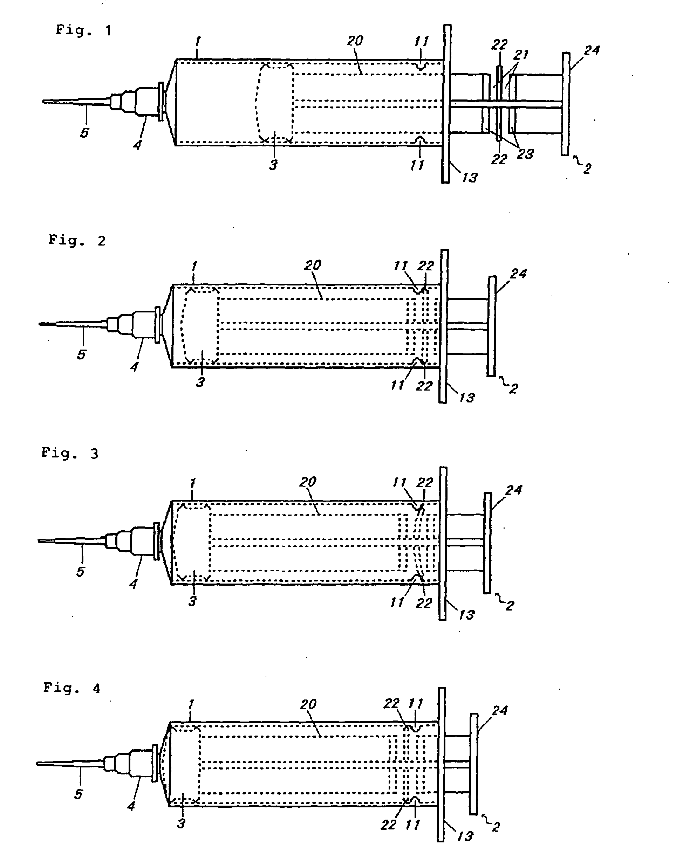

FIG. 1 is a front perspective view of the syringe in accordance with the present invention. As shown in FIG. 1, the hollow tubular barrel main body 1 has on its extremity a barrel extremity nozzle 12 for mounting the needle 5 and an external collar member 4. It further has on its base an aperture for receiving a piston 2 and a flange 13 for fingers to latch onto when an injection is carried out. There is further formed a convex annular projection 11 on the inner circumference of the barrel main body 1 on the base side.
The piston 2 is provided within the barrel main body 1, the piston 2 reciprocating therein. Set up on the extremity of the piston 2 is a gasket 3 that is in sliding contact with the inner face of the barrel main body 1 in a liquid-tight manner. Set up on the base of the piston 2 is a knob 24 for thumb/finger depressing. Furthermore, on the side of the piston 2 on the base side, a pair of plate-like projections 22 are set up in a gap 21 defined in one part of the piston 2, the projections 22 extending in a direction perpendicular to the axial direction of the syringe.
In accordance with the above-mentioned syringe constitution, the plate-like projections 22 come into contact with the annular projection 11 and snap back as the piston 2 slides when the liquid with which the syringe is filled is discharged. This generates a distinct aural signal and/or tactile signal. Means for generating each of the aforementioned signals are the plate-like projections 22 and the annular projection 11. These plate-like projections 22 and annular projection 11 are set up on the side of the piston 2 and the inner face of the barrel main body 1 respectively, which are not areas where engagement members such as the needle base and the piston extremity are positioned. This allows both the projections to have larger dimensions than those of the above-mentioned engagement members, thereby allowing a recognisable notice signal to be reliably generated.
Generation of an aural signal and/or a tactile signal by signal generating means such as the above-mentioned projections is also a characteristic of the present invention. It becomes possible thereby to easily confirm, by a distinct aural signal and/or tactile signal, the state of engagement between the needle base and the piston extremity within the extremity of the barrel main body, such an engagement being difficult to be visually confirmed in a conventional syringe.
Since the above-mentioned aural signal and/or tactile signal are generated by physical contact and snapping back of the two projections, in order to generate a distinct notice signal it is desirable for one of the two projections, which are signal generating means, to be a thin flexible plate-like projection. In this case, it is preferable for the projection that is set up on the side of the piston to be a plate-like projection. Since a plate-like projection bends due to a stress applied to its extremity, it is necessary for it to have a shape in moderate dimensions. However, if an attempt is made to provide such a shape on the inner face of the cylindrical barrel main body, it is moulded by undercutting, thereby making it comparatively difficult to mould, it being therefore desirable to provide the plate-like projection on the side of the piston.
Embodiments of the plate-like projection 22 are shown in FIGS. 9 (a) to 9 (d). FIGS. 9 (a) to 9 (d) are transverse sectional views of the piston 2 including the plate-like projection 22 at line X-X' in FIG. 7 (a). The plate-like projections 22 shown in FIGS. 9 (a) to 9 (c) are provided as a pair that extend from the piston main body 20 in opposite directions perpendicular to the axial direction of the piston. With regard to the shape of the plate-like projections 22, any shape such as a rod (FIG. 9 (a)), a trapezium (FIG. 9(b)) or an inverted trapezium (FIG. 9 (c)) can be employed to achieve the object of the present invention. The shape of the plate-like projection extremities 221 that make contact with the projection on the barrel main body 1 inner face can be any shape such as a flat shape shown in FIGS. 9 (a) and 9 (b) or an arc shape shown in FIG. 9 (c), the arc shape having a large section that is in contact with the annular projection 11 on the barrel inner face. Furthermore, the plate-like projection 22 shown in FIG. 9 (d) has a complete circular shape, and the object of the present invention can be accomplished with this shape.
The thickness of the plate-like projection 22 in the axial direction is desirably set so as to have an appropriate dimension since, in order to achieve the object of the present invention, it is necessary for the plate-like projection 22 to bend in response to the stress applied to the extremity 221 and be restored to its original shape by its resilience as soon as the stress is released, thereby generating an aural signal and/or a tactile signal. If the thickness of the plate-like projection 22 in the axial direction is too small, the resilience cannot cause a sufficiently recognisable aural signal, and if the thickness is too large, the resistance for pressing the piston becomes high due to the stress generated when the plate-like projection 22 collides with the projection on the inside of the barrel main body, thereby degrading the operability. It is therefore desirable for the thickness of the plate-like projection 22 to be approximately 1.0 ± 0.5 mm.
With regard to the plate-like projection 22 provided on the piston main body 20 perpendicular to the axial direction of the piston in the syringe of the present invention, the object of the present invention can be achieved by at least one plate-like projection 22 being provided, but the constitution as shown in the examples in which a pair thereof are set up in opposite positions so as to extend in a direction perpendicular to the axial direction of the piston can absorb a displacement in the transverse direction of the piston caused during the piston is pressed and make it possible for the plate-like projections 22 to make contact with the annular projection 11 on the inner face of the barrel main body 1, this constitution therefore being more effective.
The shape of the projection provided on the inside of the barrel main body 1 is preferably an annular structure provided on the circumference on the inner face of the barrel main body 1. The piston 2 that slides within the cylindrical barrel main body 1 has the gasket 3 set up on its extremity, the gasket 3 having a cylindrical structure in order to maintain liquid-tightness with the barrel main body 1. The piston 2 can therefore freely rotate within the barrel main body 1. A preferable mode for the projection on the inner face of the barrel main body 1 is therefore an annular projection 11 provided on the circumference on the inner face so that it can make contact with the plate-like projection 22 provided on the piston 2 regardless of their positional relationship. The cross-sectional shape of the projection is required to be a shape that can make contact with the extremity 221 of the plate-like projection 22 provided on the piston main body 20, can bend the plate-like projection 22 by the stress it applies to the plate-like projection 22, and can release the stress applied thereto as the piston is further depressed. In order to satisfy the above-mentioned requirements, the cross-sectional shape of the projection provided within the barrel main body 1 is preferably an arc shape.
With regard to the syringe of the present invention, it is preferable that completion of the injection (when the gasket comes into contact with the extremity of the barrel main body as a result of the piston sliding), engagement of the needle base with the piston extremity, and occurrence of an aural signal and/or a tactile signal are substantially simultaneous. Since the object of the present invention is to provide notification of the engagement between the needle base and the piston extremity by means of each of the signals, it is not necessary that the above-mentioned timings are strictly simultaneous. The object of the present invention can be achieved sufficiently by a syringe in which, for example, after the injection is completed and the engagement between the needle base and the piston extremity is completed, an aural signal and/or a tactile signal occur with a slight delay.
It is necessary to generate the above-mentioned aural signal and/or tactile signal at substantially the same time as the injection is completed, and it is also necessary not to interfere with the liquid-tightness and the sliding performance of the gasket 3, which slides within the barrel main body 1. With regard to the positional relationship between the aural signal and/or tactile signal generating projection 11 provided within the barrel main body 1 and projection 22 provided on the side of the piston 2, as shown in FIGS. 1 to 5, it is preferable for the projection 11 provided within the barrel main body 1 and the projection 22 provided on the side of the piston 2 to be positioned comparatively closer to the base side of the barrel main body 1 and of the piston 2. More specifically, they are preferably positioned on the base side half of the overall length of the barrel main body 1 (or the piston 2).

### EXAMPLES

FIGS. 1 to 6 are front perspective views illustrating the state of a syringe of the present invention in each of a series of stages in an injection procedure. The arrangement and the mechanism of action in each stage are explained by reference to these drawings.
FIG. 1 is a diagram showing one stage during the operation of discharging the liquid filling the syringe of the present invention. In a typical discharge operation, the barrel main body 1 is held by fingers operating the syringe, for example, an index finger and a ring finger, the two fingers being contacted with the flange 13 so as to latch onto it, the thumb is placed in contact on the knob 24 to depress it, and the gasket 3 of the piston 2 is made to slide toward the extremity side of the barrel main body 1.
When the knob 24 of the piston is pressed, the plate-like projection 22 provided on the piston 2 comes into contact with the annular projection 11 provided on the inner face of the barrel main body 1 as shown in FIG. 2.
Further pressure on the knob 24 applies a stress to the extremity 221 of the plate-like projection 22, the extremity 221 being in contact with the annular projection 11, thereby bending the plate-like projection 22 toward the piston knob 24 as shown in FIG. 3.

**[0026]** Pressing the piston knob 24 while the plate-like projection 22 is bent detaches the plate-like projection extremity 221 from the annular projection 11 it is in contact with, thereby releasing the stress. Subsequently as shown in FIG. 4, due to the resilient force the plate-like projection 22 is instantaneously restored to its original shape, that is to say, the straight shape that is perpendicular to the axial direction. At this moment, a click sound occurs, which is a distinct aural signal. Also at this moment, an instantaneous change in the load, which is a tactile signal, is generated toward the operating fingers and/or thumb. Since the piston extremity reaches the end face of the extremity of the barrel main body 1, discharge of the liquid filling the syringe is completed and at the same time the engaging means 61 on the needle base 6 engages with the engaging means 25 on the piston extremity (not illustrated).
Finally, pulling back the piston 2 to the barrel base side retracts the connected needle 5 within the barrel main body 1 as shown in FIG. 5, thereby completing the injection procedure sequence.
In the syringe in accordance with the present invention, the shape of the projection provided on the piston 2 for generating an aural signal and/or a tactile signal in order to provide notification of engagement between the needle base 6 and the piston extremity is preferably a plate-like projection set up in a direction perpendicular to the axial direction of the piston 2. FIGS. 7 (a) and (b) are a front view and a top view of the piston 2 in accordance with the present invention. As shown in FIGS. 7 (a) and (b), the piston main body 20 normally has a structure having a cross-shaped cross section, gaps 21 are provided on the base side of the piston main body 20 in opposing parts of the cross-shaped structure, and a pair of plate-like projections 22 are set up perpendicular to the axial direction of the piston in these gaps 21. Owing to this constitution, a stress generated in the extremity 221 of the plate-like projections 22 can bend the plate-like projections 22. Reinforcing members 23 are provided on opposite edges of the gaps 21. These reinforcing members 23 are moulded integrally with the piston main body 20 in order to prevent any degradation in the strength of the piston main body 20 caused by formation of the gaps 21 on opposing parts of the cross-shaped structure of the piston main body 20. The piston 2 is usually moulded by injection moulding, and while taking into consideration the mouldability, etc. during the injection moulding it is possible to form a reinforcing member 23 having a shape that surrounds the plate-like projections 22 as shown in FIGS. 8 (a) and (b).
With regard to both of the projections for generating an aural signal and/or a tactile signal (a click sound and/or a change in the load), which is an object of the syringe in accordance with the present invention, their specific shapes have been explained above. In order to form these shapes on each of the components, it is unnecessary to employ any additional special component, and they can be formed integrally with the main bodies. Moreover, they can be formed by changing parts of the shapes of components of a normal retractable-needle syringe; the number of components does not increase, it is unnecessary to change the steps in the syringe assembly, and it is possible to prevent any great increase in cost accompanying the change in shape. With regard to an injection operation, it is characterised in that the injection can be carried out by the same operation as is used for a typical retractable-needle syringe, it is unnecessary to employ any special operating procedure, and the operability is comparable to that of a normal injection operation.

The syringe in accordance with the present invention is a syringe in which the needle can be retracted within the barrel main body 1 after the injection is completed. In order to achieve this function, the syringe is provided with a mechanism for engaging the needle base 6 with the piston extremity. One embodiment of the mechanism is explained by reference to FIGS. 6 (a) and (b). FIG. 6 (a) is a cross section of the extremity side of the piston main body of a syringe in accordance with one embodiment of the present invention, showing a state in which a piston 2 is made to slide toward the needle side in order to discharge during injection the liquid filling the syringe. Referring to FIG. 6 (a), engaging means 25 for engaging with the needle base 6 extends from the piston extremity, penetrates a gasket 3 and opens at the end thereof so as to engage with engaging means 61 of the needle base 6. A needle 5 is connected to one end of a barrel extremity nozzle 12, and the needle base 6 having the engaging means 61 is provided so as to be in internal contact with the barrel extremity nozzle 12 at the other end, and is prevented from moving toward the barrel base side by a ridge 62. Furthermore, an external collar member 4 is mounted around the barrel extremity nozzle 12 so as to be in contact with the needle base 6, thereby preventing it from accidentally projecting outward.
FIG. 6 (b) illustrates a state in which the piston 2 has reached the extremity of the barrel main body 1. As shown in FIG. 6 (b), making the piston 2 slide within the barrel main body 1 and move toward the needle 5 side allows the gasket 3 to make contact with the end face of the extremity of the barrel main body 1 when the discharge operation is completed. The engaging means 61 extending from the needle base 6 is thereupon fitted into and engaged with the engaging means 25 set up on the piston extremity and opening so as to receive the engaging means 61. The connection between the needle base 6 to which the needle 5 is connected and the piston 2 is thereby completed. Furthermore, when the engaging means 61 is fitted into and engaged with the engaging means 25, the engaging means 61 is released from the contact with the inner surface of the barrel extremity nozzle 12 as shown in FIG. 6 (b), thereby allowing the needle 5 to move toward the piston base side.
One embodiment of the mechanism for engaging the needle base 6 with the piston extremity is explained above by reference to FIGS. 6 (a) and (b), but the present invention is in no way limited by this embodiment.

### [Effect of the Invention]

In accordance with a characteristic of the syringe of the present invention, with regard to a syringe that enables a needle to be retracted within a barrel main body on completion of an injection by engaging a needle base with an extremity of a piston and pulling back the piston, by providing projections in positions such as the inner face of the barrel main body and the side of the piston, which are positions other than those of the respective engagement means, on completion of the injection substantially simultaneously with engagement, of engagement means provided on the piston extremity and engagement means extending from the needle base to which the needle is connected, the projection provided on the inner face of the barrel main body and the projection provided on the side of the piston make physical contact, a projection bends due to stress applied thereon and its original shape is restored instantaneously on release of the stress, thereby generating a distinct aural signal and/or a tactile signal (a click sound and/or a change in load). It is therefore achieved that audible and/or tactile recognition of the engagement is easily and reliably carried out with respect to the piston extremity and the needle base to which the needle is connected by means of an aural signal and/or a tactile signal generated on completion of the injection, and the subsequent pulling-back operation to retract the needle within the barrel main body can thus be initiated assuredly and reliably.

## Claims

1. A syringe that allows a needle (5) to be retracted within a barrel main body (1), on completion of the discharge of a liquid filling the syringe, by engaging a needle base (6) provided on an extremity of the syringe with an extremity of the piston (2) and pulling back the piston (2),
**characterized in that**
signal generating means for generating a notice signal to provide a notification of completion of the engagement between the needle base (6) and the extremity of the piston (2) is provided as projections (11, 22) provided on the inner face of the barrel main body (1) and on the side of the piston (2), and the notice signal is generated by physical contact of the two projections accompanying sliding of the piston (2).

2. The syringe according to Claim 1, wherein the projection provided on the inner face of the barrel main body (1) is an annular projection (11) formed integrally on the inner periphery of the barrel main body (1).

3. The syringe according to Claim 1 or Claim 2, wherein the projection provided on the side of the piston (2) is at least one plate-like projection (22) provided perpendicular to the axial direction of the piston (2).

## Patentansprüche

1. Spritze, welche es einer Nadel (5) ermöglicht, in einen Trommelhauptkörper (1) bei Vervollständigung des Austrags einer flüssigen Füllung der Spritze zurückgezogen zu sein bzw. zu werden, indem eine Nadelbasis (6), die an einem Ende der Spritze zur Verfügung gestellt ist, mit einem Ende des Kolbens (2) ergriffen wird, und der Kolben (2) zurückgezogen wird,
**dadurch gekennzeichnet, daß**
Signalerzeugungsmittel zum Erzeugen bzw. Generieren eines Hinweissignals zum Bereitstellen einer Mitteilung bzw. Verständigung bzw. Notifikation einer Vervollständigung des Eingriffs zwischen der Nadelbasis (6) und dem Ende des Kolbens (2) als Vorsprünge bzw. Erhebungen (11, 22) zur Verfügung gestellt sind, die an der Innenfläche des Trommelhauptkörpers (1) und an der Seite des Kolbens (2) zur Verfügung gestellt sind, und das Hinweissignal durch einen physikalischen Kontakt der zwei Vorsprünge generiert ist, der ein Gleiten des Kolbens (2) begleitet.

2. Spritze nach Anspruch 1, wobei der Vorsprung, der an der Innenfläche bzw. -seite des Trommelhauptkörpers (1) zur Verfügung gestellt ist, ein ringförmiger Vorsprung (11) ist, der integral bzw. einstückig an dem Innenumfang des Trommelhauptkörpers (1) ausgebildet ist.

3. Spritze nach Anspruch 1 oder 2, wobei der Vorsprung, der an der Seite des Kolbens (2) zur Verfügung gestellt ist, wenigstens ein plattenartiger Vorsprung (22) ist, der senkrecht zu der axialen Richtung des Kolbens (2) zur Verfügung gestellt ist.

## Revendications

1. Seringue qui permet de rétracter une aiguille (5) dans un corps principal tubulaire (1), à la fin de l'éjection d'un liquide remplissant la seringue, par enclenchement d'une base d'aiguille (6) prévue sur une extrémité de la seringue avec une extrémité du piston (2) et rétraction du piston (2),
**caractérisée en ce que**
des moyens de génération de signal, pour engendrer un signal indicatif fournissant une notification d'achèvement de l'accouplement entre la base d'aiguille (6) et l'extrémité du piston (2), sont prévus sous la forme de saillies (11, 22) situées sur la face intérieure du corps principal tubulaire (1) et sur le côté du piston (2), et le signal indicatif est engendré par contact physique des deux saillies, accompagnant le coulissement du piston (2).

2. Seringue selon la revendication 1, dans laquelle la saillie prévue sur la face intérieure du corps principal tubulaire (1) est une saillie annulaire (11) formée solidairement sur la périphérie intérieure du corps principal tubulaire (1).

3. Seringue selon la revendication 1 ou la revendication 2, dans laquelle la saillie prévue sur le côté du piston (2) est au moins une saillie en forme de plaque (22) perpendiculaire à la direction axiale du piston (2).
